Europäisches Patentamt

European Patent Office

Office européen des brevets

(11) Publication number: **0 233 014**
**A2**

## EUROPEAN PATENT APPLICATION

(21) Application number: **87300768.6**

(22) Date of filing: **29.01.87**

(51) Int. Cl.4: **C 08 F 291/00**
**C 08 F 265/02, A 61 L 15/00**

(30) Priority: **04.02.86 GB 8602649**

(43) Date of publication of application:
**19.08.87 Bulletin 87/34**

(84) Designated Contracting States:
**BE DE ES FR GB IT NL SE**

(71) Applicant: **ALLIED COLLOIDS LIMITED**
**P.O. Box 38 Low Moor**
**Bradford West Yorkshire, BD12 0JZ (GB)**

(72) Inventor: **Flesher, Peter**
**"Little Beck" Beck Lane**
**Bingley West Yorkshire (GB)**

**Farrar, David**
**13 Greenfield Lane Idle**
**Bradford West Yorkshire (GB)**

(74) Representative: **Lawrence, Peter Robin Broughton et al**
**GILL JENNINGS & EVERY 53-64 Chancery Lane**
**London WC2A 1HN (GB)**

(54) Polymers and processes for their production from water soluble monomers.

(57) Water soluble or water swellable polymeric material is made by forming a dispersion in a non-aqueous liquid of water absorbent seed particles formed from first monomer, absorbing an aqueous solution of water soluble ethylenically unsaturated second monomer into the particles and polymerising the second monomer to form the said polymeric material. The invention is of particular value in the production of irregularly shaped non-spheroidal highly absorbent polymeric particles suitable for use in diapers, or for the production of soluble polymers.

## Description

### Polymers and Processes for Their Production from Water Soluble Monomers

Substantially dry, relatively large, particles of water soluble polymers or water insoluble but water swellable polymers are usually at present made commercially by gel polymerisation or by reverse phase bead polymerisation.

In gel polymerisation an aqueous solution of monomer is polymerised to form a gel which is then comminuted and dried. This technique is not very satisfactory for some polymers. For instance particular problems arise from the requirement that the amount of water in the monomer solution should be relatively low. This is usually essential since the relevant monomers usually polymerise exothermically and so if the concentration of monomer is too high the polymerisation exotherm will result in the polymerisation becoming uncontrolled, and possibly dangerous. It is therefore inevitable that the gel always contains a substantial amount of water and removal of this after comminution involves considerable heat energy. If the molecular weight of the gel is relatively low, e.g., below l million, the gel is generally too soft to be comminuted satisfactorily. Comminution is generally conducted with the intention of forming an average particle size typically in the range l00 µm to 800 µm but results also in the production of a significant proportion of fines and with some gels the proportion of fines is unacceptably high. For instance highly ionic cross linked gels are difficult to comminute. The removal of fines is desirable in order to avoid dusting. Some gels tend to aggregate seriously during drying, after comminution.

Reverse phase bead polymerisation involves dispersing aqueous monomer in a non-aqueous liquid in the presence of a dispersion stabiliser and then initiating polymerisation to form beads of polymer gel. The product may be dried by azeotroping and the beads separated from the non-aqueous liquid. Again the average particle size is generally intended to be in the range l00 to 800 µm. The beads are essentially spheroidal but it is difficult to achieve wholly satisfactory stabilisation during the process and so the final product is generally a blend of spheroidal particles with broken or mis-shapen, for instance pear-shaped, particles or fines. This occurs seriously with low molecular polymers. Although it is intended that the particle size range should be narrow some of the liquid particles merge during polymerisation to form large, substantially spheroidal, particles. The process tends to be more satisfactory for high molecular weight water soluble polymers than for water swellable polymers or for low molecular weight soluble polymers, whose beads tend to collapse during the process.

Despite the presence of mis-shapen or broken beads, the majority of the beads have a regular, generally substantially spherical, shape and often a shiny surface. Whilst this is very desirable for some purposes it is less satisfactory for others. For instance if the particles are incorporated into fibrous articles such as bandages or disposable diapers these spherical shiny beads tend to migrate out of the articles.

Despite the difficulties, it is generally recognised that gel polymerisation and reverse phase bead polymerisation are the best commercial ways of making dry particles of water soluble or swellable polymers. However a number of improvements in these processes would clearly be desirable.

Thus it would be desirable to be able to make the gel to a much higher polymer concentration without risk of difficulties from the polymerisation exotherm, in order that the heat energy required for drying the gel is reduced and/or the gel can have a more rigid and comminutable structure. In particular it would be desirable to be able to make a gel product of low molecular weight polymer either in a comminutable form, despite its low molecular weight, or in the form of particles that can be of controllable size, in contrast to the tendency of low molecular weight beads to collapse during reverse phase bead polymerisation. It would be desirable to be able to make absorbent particles by a process that involves the benefits of reverse phase bead polymerisation but that does not result in the production of shiny beads that tend to migrate out of diapers or other absorbent articles.

In DE 3429379 a process is described in which a carboxylic containing resin such as sodium polyacrylate is formed by polymerisation while emulsified in cyclohexane and is dehydrated, in the presence of, e.g., about 2.2% (based on aqueous monomer) emulsifier. Aqueous hydroxyethyl acrylate with cross linking agent, or other reactive monomer or a polymer having reactive groups, is then added to a dispersion of the sodium polyacrylate particles in cyclohexane and polymerisation or cross linking is induced, so as to give what appears to be a very small core of sodium polyacrylate with a very thin shell of a different polymer, that can then react with, for instance, an isocyanate prepolymer to form a polyurethane film having sodium polyacrylate particles encapsulated within it. Because of the large amount of emulsifier these particles will inevitably be very small and this process is of no value for meeting any of the problems described above. It is limited to the production of a core-shell system in which the core and shell are formed of different polymeric materials and the shell reacts into a polyurethane film.

For most purposes it is desirable for water absorbent or water soluble polymeric materials to consist of a single polymeric type or to consist of two types of which at least 50% by weight are formed from the same monomer. There is, however, one situation where it is desirable to have different polymer types in the same polymeric material and this is in the production of organic coagulants, generally low molecular weight cationic polymers, in combination with other cationic polymers that can also be coagulants or can be high molecular weight flocculants. It is often desired to administer, for instance, a coagulant and a flocculant at the same point of application and it would be desirable to supply them as a single composition. Unfortunately they tend to be incompatible and so normally have to be supplied separately.

In Polish patent 101002 a powdered substrate which can be powdered polysodium acrylate is sprayed with an aqueous solution of polymerisable monomer that can be sodium acrylate and which is then polymerised while the particles are, apparently, exposed to air, and the resultant product is in the form of granules that can be separated by sifting. This process is not very reproducible as regards degree of polymerisation and particle size and so is unsatisfactory for commercial purposes.

It is of course well known to use seed polymerisation of water insoluble monomers, such as styrene or methyl methacrylate, to form polymers that neither swell nor dissolve in water. Thus in U.S. 4,459,378, 4,530,956 and 4,564,644 seed polymer particles that are insoluble and non-swellable in water are impregnated with a water insoluble monomer which is then polymerised. The purpose is to produce particles having a shell-core structure and/or to produce particles of a very regular small particle size and shape, e.g., as a component of an aqueous latex. In each instance it is always intended that the particles shall be used as such, generally dispersed in a continuous liquid phase. Again therefore these techniques are of no value for solving the problems that arise in the production of water soluble and water swellable polymers.

In the invention we make water soluble or water swellable polymeric material by a method comprising forming a dispersion in non-aqueous liquid of water absorbent seed particles formed from first monomer, absorbing an aqueous solution of water soluble ethylenically unsaturated second monomer into the particles and polymerising the second monomer to form the said polymeric material in the form of particles which preferably have an average dry particle size of at least 100μm.

Typically at least 90% by weight are above 100μm and usually at least 90% are above 200μm but below 700μm. The average size is usually above 200μm. It can be up to 200μm but is usually below 1000μm (dry size). These particles may be monoparticles or they may be aggregates of several particles.

Although the process can be used to produce monoparticulate, regular beads, it is normally conducted to produce polymeric material that is wholly or mainly in the form of aggregates or of irregularly shaped non-spheroidal particles and the product is normally dried and is then crushed. This is entirely contrary to the normal purposes of seed polymerisation which is to produce very regular shaped particles which are used as such.

The irregular particles of polymeric material that are formed either by the polymerisation in the non-aqueous liquid or that are formed by crushing the product of such polymerisation are of particular value for incorporation in absorbent articles comprising an absorbent fibrous body in which the particles are physically trapped. For instance the irregular particles of water insoluble, water swellable polymeric material may be physically trapped in the absorbent fibrous body of a diaper, catamenial device or bandage. The irregular shape minimises the tendency for the particles to escape from the fibrous body.

The process can be used to form two different polymer types (e.g., a coagulant-flocculant blend as described below) but preferably the first monomer is water soluble ethylenically unsaturated monomer (or monomer blend) of which at least 50% by weight is substantially identical to at least 50% by weight of the second monomer. For instance at least 50% by weight of the first monomer may be acrylic acid or a water soluble salt thereof in which case at least 50% by weight of the second monomer should also be of acrylic acid or a water soluble salt thereof. The monomers should be substantially identical in the sense that they are of the same monomer type but it is not essential that the same form of acrylic acid is used in both the first and second monomers. Thus one type of salt could be present in the first monomer and a different type in the second. The use of the same monomer as part or all of the first and second monomers is in contrast to DE 3429379 where different monomers are essential.

One preferred product of the invention is a water soluble polymeric material, preferably having an average size above 100μm (dry size). This is in contrast to the products of normal seed polymerisations, where it is always desired to produce particles that are not dissolved but have a distinct particle shape that is retained during use. In practice the soluble polymers of the invention are dissolved in water and are used as, for instance, coagulants or flocculants in, e.g., clarifying or dewatering aqueous suspensions or as retention aids in the production of paper.

Another preferred product of the invention is a water insoluble but water swellable polymeric material that is produced in the non-aqueous liquid wholly or mainly in the form of agglomerates or of irregularly shaped non-spheroidal particles that have an average size above 100μm (dry size). This is in contrast to conventional bead polymerisation and seed polymerisation which are always conducted with the intention of producing a product that consists substantially only of monoparticles that are as near spheroidal as can be achieved.

The seed particles can include some fines, e.g., below 50μm dry size, but it is preferred that they have an average dry size above 50μm and most preferably above 100μm, usually at least 80% in the range 100 to 300μm. The average size is preferably below 500μm, preferably below 300μm. They can be substantially spheroidal, for instance being the product of a bead polymerisation process, but often they are irregularly shaped, crushed, particles.

It is then relatively easy to operate the polymerisation process in the non-aqueous liquid such that the final polymeric material that is produced in the non-aqueous liquid has a corresponding irregular shape that is an enlargement of the shape of the seed particles. This is best achieved by arranging for the aqueous second monomer to be absorbed substantially entirely into the seed particles before initiating polymerisation.

When, instead of monoparticles, it is desired to achieve a product that is primarily in the form of agglomerates this is best achieved by causing polymerisation of the second monomer primarily at the surfaces of the seed particles. Thus agglomerates can be formed by polymerising aqueous second monomer impregnated into the surface of seed particles of water swellable cross linked polymer while allowing aggregation of the seed particles, and drying the resultant aggregates. Polymerisation primarily at the surface of the particles can be achieved by initiating polymerisation quickly after starting impregnation of the monomer into the particles, before the monomer has had an opportunity to impregnate homogeneously throughout the particles. Absorption of the second monomer into the seed particles can be inhibited, and agglomerate production increased, by using seed particles that are coated with a hydrophobic material. This may be residue of a lubricant that was used for lubricating the comminution of gel particles or it may be the residue of a surfactant or amphipathic stabiliser that was used during bead polymerisation of bead particles.

It is possible to disperse dry seed particles into an emulsion of aqueous second monomer in non-aqueous liquid or to add concentrated second monomer to a dispersion in non-aqueous liquid of seed particles that are swollen by some water. Preferably however the process is conducted by dispersing dry seed particles into non-aqueous liquid and then mixing into the dispersion aqueous second monomer, which will thereby become absorbed by the seed particles. These seed particles must be absorbent such that they can absorb the aqueous monomer and they can be either water soluble or water insoluble but swellable.

Suitable polymerisation initiator must be included in the dispersion, usually in the aqueous solution of second monomer. Any of the conventional initiators suitable for the polymeristaion of the second material may be used, including redox, thermal, or ultra-violet with photosensitiser initiator systems. Radiation polymerisation may be used.

The non-aqueous liquid can be any of the non-aqueous liquids conventionally used for reverse phase polymerisation systems. It is generally desirable to include a stabiliser in order to promote the distribution of the aqueous monomer throughout the non-aqueous phase and/or to promote stability of the dispersion during polymerisation and/or dehydration. The use of both, and the use of more than about 1% (based on the weight of aqueous second monomer) or at the most 2%, emulsifier, is undesirable as it tends to promote the formation of fines. An emulsifier is generally unnecessary, or even undesirable.

Suitable stabilisers include oil soluble dispersion stabilisers such as an amphipathic stabilisers of the type conventionally used for stabilising reverse phase bead polymerisation dispersions, e.g., stearyl methacrylate-methacrylic acid or -dimethylaminoethyl methacrylate copolymers. When the polymeric material is cationic, the stabiliser should be cationic. Suitable stabilisers and non-aqueous liquids may be any of those described in the literature for reverse phase bead polymerisation processes, for instance as described in EP 126528. The dispersion may be stirred during polymerisation, but too much stirring may be undesirable and lead to excessive formation of fines.

Although it may be possible to use the polymeric material without preliminary drying it is normally preferred to dry the polymeric material and separate it from the non-aqueous liquid. Thus the polymeric material is generally dried by azeotroping in conventional manner and then separated from residual non-aqueous liquid, e.g., by centrifuging in conventional manner.

If desired the final particles may be comminuted before or after drying and before or after separating from the non-aqueous liquid. The final particles generally have dry average diameters in the range 50 to 2,000 micron, preferably 100 to 1000 micron, preferably 90% of the particles have diameters in the range 200 to 700 micron. Any final drying may be by conventional methods, for instance fluid bed drying.

The second monomer is always a water soluble ethylenically unsaturated monomer, and may be a blend of such monomers or a blend of water soluble and water insoluble monomers, provided the blend is itself water soluble. The second monomer is usually introduced as a 20 to 70%, preferably 30 to 55%, by weight aqueous solution.

The monomers may be cationic, anionic or non-ionic. Suitable cationic monomers are diallyl dimethyl ammonium chloride (DADMAC), dialkyl amino alkyl (meth) acrylates, generally as acid addition or quaternary ammonium salts, or dialkyl amino alkyl (meth) acrylamides as free base, acid addition or quaternary ammonium salts. Any cationic monomers may be used alone but preferably as a blend with 10 to 90% by weight non-ionic monomer, generally acrylamide.

Anionic monomers may be free carboxylic acids or free sulphonic acids but are usually present as salts with alkali, usually sodium or potassium, or ammonia. Dicarboxylic monomers may be present as anhydrides. Suitable carboxylic monomers are acrylates, methacrylates, itaconates and crotonates, preferably acrylate. Suitable sulphonic acids include allyl sulphonic acid and 2-acrylamido-2-methyl propane sulphonic acid. The polymerisable anionic monomers may be copolymerised with comonomers. Such comonomers are usually included in amounts of less than 50% by weight based on the total monomer weight, preferably less than 50% by weight based on the total monomer weight, preferably less than 25% and most preferably less than 10%. The comonomer is generally non-ionic.

Suitable non-ionic monomers include acrylamide, methacrylamide and N-substituted acrylamides, and lower alkyl and hydoxy alkyl acrylates such as hydroxy ethyl acrylate or methacrylate.

The seed polymer particles must be formed from monomer such that aqueous second monomer is absorbed by them. They are often formed from monomers selected from those mentioned above or, in some instances, they may be formed from other monomers and may be, for instance, polyethylene imine or polyamine-epichlordryin condensation products.

One aspect of the invention involves its application to the production of polymeric material that is water insoluble but water swellable and is dried and is separated from the non-aqueous liquid in the form of agglomerates or irregularly shaped non-spheroidal particles of above $100\mu m$ average dry size. In the production of such particles the polymeric material is preferably a polymer of acrylic acid or other unsaturated carboxylic acid, preferably the homopolymer, but sometimes as a copolymer with a minor amount of a comonomer such as acrylamide, preferably in an amount of less than 50% by weight, more preferably less than 20% by weight of the total monomer charge. The acrylic acid is generally at least partially neutralised, for example at least 50% neutralised more preferably above 60% neutralised, and often less than 90% neutralised. The acrylate is generally the sodium or the ammonium salt or a mixture of the two. Generally the same monomer or monomer blend is used for the seed polymer as the second monomer but one type of salt could be used in the second monomer and another used for the production of the seed polymer.

It seems that grafting or other cross linking probably occurs during the polymerisation of the second monomer on to the seed polymer, especially when the seed polymer is of acrylic acid or other ethylenically unsaturated carboxylic acid, and so we find surprisingly that water insoluble but water swellable polymer particles can be formed satisfactorily even if either or both of the first and second monomers are free of cross linking agent. However it is generally preferred that either, and usually both, monomers include cross linking agent. Preferably the cross-linking reagent comprises a polyfunctional copolymerisable ethylenically unsaturated monomer, generally a difunctional monomer, but polyvalent metal or other cross linkers may be used. The amount of the reagent is usually more than 10 ppm, often more than 50 ppm. Usually the amount is below 3000 ppm, and often below 1000 ppm. Best results are generally achieved at values of 100 to 500 ppm. In general any of the cross-linking agents suitable for cross-linking polymers formed from water-soluble monomers may be used.

Examples of suitable polyfunctional compounds include divinyl compounds such as divinyl benzene and divinyl diethylene glycol diether; allyl compounds such as allyl methacrylate, allyl acrylate, diallyl phthalate and diallyl sucrose; polyfunctional acylates and methacrylates such as glycol diacrylate, glycol dimethacrylate, pentaethritol tetra-acrylate and trimethylol propane trimethacrylate; polyfunctional acrylamides and methacrylamides such as N,N' methylene bisacrylamide; M-methylolacrylamide, poly-N-methylol acrylamide, glycidyl acrylate, glycidyl methacrylate, polyolpolyglycidal ethers such as ethylene glycol diglycidyl ether, and epichlorohdrin The preferred cross-linking agent is N,N'-methylene bisacrylamide.

The particles may be provided with a surface layer of reduced swellability, generally by cross linking, in order to minimise aggregation and water absorption properties during use. Thus the particles may be subjected to surface cross linking, generally by contacting them with a polyvalent metal salt which preferably is present as a polyvalent metal compound that is soluble in the non-aqueous liquid, for instance an aluminium alkoxide. Preferably this is introduced before the polymerisation of the second monomer but can be introduced after polymerisation of the second monomer and before azeotropic distillation.

A second aspect of the invention provides the final polymeric material as a material that is soluble in water. It is particularly preferred that the seed particles or the polymer formed from the second monomer, or both, should have molecular weight below 1 million. Such processes offer a very convenient way of avoiding the disadvantages of known methods of making solid grade products having a relatively low molecular weight component.

The process is of particular value when the entire polymeric material, i.e., both the seed particle polymer and the polymer formed from the second monomer, has molecular weight below 1 million. Thus a low molecular weight polymer that normally has a gel structure that is unsatisfactory for normal gel or suspension polymerisation can be made by the seed polymerisation method of the invention. The polymeric material is produced in the form of gel that can have a very high polymer content, e.g., above 40 or 50% by weight and up to 70%, 80% or even higher without the problems that are normally encountered due to the polymerisation exotherm since some, usually 30 to 60%, of the final polymer is present initially as seed polymer. For instance typical proportions are 1 part seed polymer, 0.8 to 2 parts monomer and 0.8 to 1.5 parts water. Also it is possible to obtain higher molecular weights than is obtainable by conventional polymerisations.

The low molecular weight polymers are preferably polymers formed from first and second monomers each of which comprise at least 20%, and generally at least 50%, by weight water soluble ethylenically unsaturated ionic monomer wherein the ionic monomer is substantially the same in each and is selected from DADMAC, dialkylaminoalkyl (meth) -acrylates and -acrylamides and their salts, and ethylenically unsaturated carboxylic and sulphonic acids and salts. Any remaining monomer is generally non-ionic.

When the polymeric material is to be a dispersing agent the monomers are preferably of ethylenically unsaturated carboxylic or sulphonic acids (generally acrylic acid or a salt and/or 2-acrylamido-2-methyl propane sulphonic acid) optionally with less than 50% acrylamide and/or a Cl-4 alkyl acrylate such as butyl acrylate that is present in a sufficiently low amount that it can dissolve into the aqueous monomer solution. Polymerisation is usually conducted in the presence of isopropanol or other molecular weight regular so as to hold molecular weight generally below 100,000, usually below 20,000.

The invention is, however, of particular value for the production of cationic polymers in which the monomers are dialkylaminoalkyl (meth) acrylate or -acrylamide, optionally copolymerised with acrylamide, or, especially, DADMAC optionally copolymerised with acrylamide. The amount of acrylamide is generally below 50% by weight. The polymeric material is preferably DADMAC homopolymer. By the invention it is possible to obtain a solid grade polymer having good physical properties and, in particular, to obtain a DADMAC polymer having a higher molecular weight than is conveniently achievable by any other polymerisation technique and in a fast process. For instance DADMAC polymers are usually of intrinsic viscosity below 0.5 dl/g and usually have to be made very dilute (to avoid the exotherm) in a process that typically takes more than 1.5 hours. In the invention they can be made more concentrated, to a higher IV, typically 0.5 to 1 or more, much faster, typically 10 to 30 minutes.

The invention is also of value when the seed polymer is a cationic polymer selected from polyethylene imine, polyamine-epichlorhydrin polymers and polymers of cationic ethylenically unsaturated monomer, and the second monomer is also cationic ethylenically unsaturated monomer. Such monomers are preferably DADMAC and dialkylaminoalkyl (meth) -acrylates and acrylamides and their salts, all optionally blended with up to 90% by weight acrylamide. The seed polymer conveniently is a relatively low molecular weight polymer, e.g., having molecular weight below 1 million, and may thus serve as a coagulant and the second monomer can be polymerised on to it either to a relatively low molecular weight or to a high molecular weight. The second monomer is generally chemically different from the first. For instance the seed polymer can be polyethylene imine, polyamine epichlorhydrin or poly DADMAC coagulant and the second monomer can be dialkylaminoalkyl (meth) -acrylate or -acrylamide, generally as a salt. Alternatively the high molecular weight polymer may be used as the seed and a cationic second monomer polymerised on to it, generally to a low molecular weight. By these techniques a blended, and possibly chemically grafted, polymeric material is produced having the characteristics both of the seed polymer and of the polymer formed from the second material, e.g., as a combined coagulant and flocculant.

When the first and second polymers are intended to be chemically different the proportions of seed polymer and second monomer will be chosen having regard to the proportions of the two polymers desired in the end product, often in the ratio 0.1 to 5 parts second monomer per part seed polymer. However in general, in the invention, it is desirable to use at least 0.5 and often at least 0.8 parts second monomer per part seed polymer. The amount of second monomer should usually be below 5 parts and usually below 3 parts as the use of increasing amounts of second monomer increases the tendency for the particles to agglomerate unacceptably.

Although we refer throughout to the use of seed particles and impregnation of them it is not essential for them to retain their identity. It is possible that in some processes they may break down during the impregnation into smaller units, and particles then reform during the polymerisation of the impregnated monomer.

The following are examples.

## Example 1

45 g of dry, methylene bis acrylamide cross linked acrylic acid polymer prepared by bulk gel polymerisation and neutralised with 50/50 $NH_4+/Na+$ and having a crushed particle size 125-300 μm was dispersed in 300 g of a hydrocarbon solvent containing 2000 ppm of a 2/1 stearyl methacrylate/methacrylic acid copolymer. The resultant dispersion was deoxygenated by nitrogen blowing and a deoxygenated aqueous solution of 135 g 35% sodium acrylate containing 75 ppm methylene bis acrylamide (solid on solid) added. The resultant, slightly viscous dispersion, was initiated with 10 ppm tertiary butyl hydroperoxide and 37 ppm sulphite added as sodium sulphite.

The resultant slurry was azeotroped to remove water and the solid filtered off and dried. The final product was a white solid which took up 60.27 g 0.9% NaCl solution per gram of polymer. It was in the form of particles having substantially the same crushed, irregular, shape as the starting seed particles and substantially all having a dry size > 100μm.

## Example 2

The process of Example 1 was repeated using seed having a size range of 63 to 110μm. The product had an absorbency of 45 to 50 g/g 0.9% sodium chloride and a dry particle size of 67% below 600μm, 17% below 500μm and 1% below 300μm. The material above 600μm was mainly aggregates and the monoparticles below 600μm were irregularly shaped.

When the seed had a size range of 150 to 250μm 61% of the product was below 600μm, 21% below 500μm and 3% below 300μm.

The process of this example was repeated with ratios of seed:monomer up to 1:3 but at the higher ratios the proportion of fines in the final product was increased.

The dried polymeric material could be used as such but preferably was crushed after drying to a substantially uniform particle size preferably in the range 100 to 300μm.

## Example 3

The process of Example 1 was repeated but the seed particles and the second monomer were free of methylene bis acrylamide, the polymeric stabiliser was omitted, and aluminium isopropoxide was added to the dispersion before azeotroping. 60% of the dry product was in the form of aggregates above 600μm.

## Example 4

60% aqueous DADMAC solution having pH 10 was dispersed in a hydrocarbon solvent in an amount of 150 grammes DADMAC to 300 grammes solvent. Cationic amphipathic polymeric stabiliser and conventional DADMAC polymerisation initiator systems were included. Polymerisation was conducted in conventional manner and it was found that the beads aggregated during polymerisation so that the product had to be recovered by acetone extraction followed by drying and grinding. It had IV 0.29 dl/g.

50 grammes of this ground product was dispersed in 300 grammes solvent and then 83.3 grammes of DADMAC (as a 60% aqueous solution having pH 10) was added with the same initiators and stabiliser as in the production of the seed polymer. The polymerisation proceeded smoothly with very little aggregate formation. The product was subjected to azeotropic distillation to remove water, followed by filtration and drying. The resultant polymeric material was a free flowing particulate material having a dry size > 100μm and that did not need grinding and that had intrinsic viscosity of 0.98 dl/g.

In a similar manner, the process of this example can be repeated but using an equivalent amount of aqueous dimethylaminoethyl acrylate methyl chloride quaternary salt instead of the 83.3 g DADMAC monomer, to form a product that is a stable soluble blend of low molecular weight DADMAC polymer and higher molecular weight cationic acrylate polymer.

**Claims**

1. A process for making water soluble or water swellable polymeric material comprising forming a dispersion in non-aqueous liquid of water absorbent seed particles formed from first monomer, absorbing an aqueous solution of water soluble ethylenically unsaturated second monomer into the particles and polymerising the second monomer to form the said polymeric material, and in which the polymeric material produced by the polymerisation in the non-aqueous liquid is in the form of particles having an average dry size of above 100μm.

2. A process according to claim 1 in which the first monomer is water soluble ethylenically unsaturated monomer of which at least 50% by weight is substantially identical to at least 50% by weight of the second monomer.

3. A process according to claim 1 in which the seed particles are irregularly shaped, crushed, gel particles.

4. A process according to claim 1 or claim 2 in which the polymeric material is dried and crushed.

5. A process according to any preceding claim in which the seed particles hve a dry average size of at least 50μm.

6. A process according to any preceding claim in which the polymeric material is water insoluble but water swellable and is produced in the non-aqueous liquid wholly or mainly in the form of agglomerates or of irregularly shaped non-spherical particles which are dried and separated from the non-aqueous liquid.

7. A process according to claim 6 in which the first and second monomers each comprise 50 to 100% acrylic acid or a salt thereof and 0 to 50% by weight acrylamide.

8. A process according to claim 6 in which the first and second monomers are each selected from acrylic acid and ammonium and alkali metal salts of acrylic acid.

9. A process according to any of claims 6 to 8 in which the first and second monomers each include cross linking agent.

10. A process according to any of claims 6 to 8 in which the seed particles have a hydrophobic coating that reduces absorption.

11. A process according to any of claims 1 to 5 in which the polymeric material is soluble in water.

12. A process according to claim 11 in which the polymer of the seed particles and/or the polymer formed from the second monomer has molecular weight below 1 million.

13. A process according to claim 11 in which the polymer of the seed particles and the polymer formed from the second monomer has molecular weight below 1 million and the first and second monomers each comprise at least 20% by weight water soluble ethylenically unsaturated ionic monomer and the ionic monomer in each is substantially the same and is selected from diallyldimethyl ammonium chloride, dialkylaminoalkyl (meth) -acrylate or -acrylamide or a salt thereof, ethylenically unsaturated carboxylic acids and water soluble salts thereof and ethylenically unsaturated sulphonic acids and water soluble salts thereof.

14. A process according to claim 13 in which the first and second monomers each consist of 50 to 100% by weight diallyldimethyl ammonium chloride and 0 to 50% acrylamide.

15. A process according to claim 11 or claim 12 in which the seed polymer is a cationic polymer selected from polyethylene imine, polyamine epichlorhydrin reaction products and polymers of cationic ethylenically unsaturated monomers and the second monomers are selected from cationic ethylenically unsaturated monomers, wherein the cationic ethylenically unsaturated monomers are selected from diallyldimethyl ammonium chloride and dialkylaminoalkyl (meth) acrylates and salts thereof.

16. A process according to claim 15 in which the seed polymer has a molecular weight of below 1 million.

17. A aqueous solution of a polymer according to any of claims 11 to 15.

18. An absorbent article comprising an absorbent fibrous body in which water absorbent particles are physically trapped, characterised in that the particles are in the form of agglomerates or of irregularly shaped non-spheroidal particles and have been made by a process according to any of claims 6 to 10.